# FASCICULE DE BREVET EUROPEEN

(11) **EP 4 127 669 B1**
(45) Date de publication et mention de la délivrance du brevet: **13.05.2026**
(21) Numéro de dépôt: 21718513.1
(22) Date de dépôt: 26.03.2021
(51) Int. Cl.: G01N 21/45, C12M 1/34, G02B 21/00

(54) **PROCEDE DE CARACTERISATION D'UN MICRO-TISSU BIOLOGIQUE PAR IMAGERIE**
VERFAHREN ZUR CHARAKTERISIERUNG EINES BIOLOGISCHEN MIKROGEWEBES MITTELS BILDGEBUNG
METHOD FOR CHARACTERISING A BIOLOGICAL MICRO TISSUE USING IMAGING

(30) Priorité: 27.03.2020 FR 2003036
(43) Date de publication de la demande: 08.02.2023
(73) Titulaire: Treefrog Therapeutics, 33600 Pessac (FR)
(72) Inventeur: ALESSANDRI, Kévin, Pascal, Stéphane, 33000 BORDEAUX (FR); BON, Pierre, 33140 VILLENAVE D'ORNON (FR)
(74) Mandataire: Aquinov
(86) Numéro de dépôt international: PCT/EP2021/057977
(87) Numéro de publication internationale: WO 2021/191433

(56) Documents cités:
- FR-A1- 3 059 009
- US-A1- 2014 375 792
- US-A1- 2018 113 064
- US-A1- 2020 096 941
- LEDWIG PATRICK ET AL: "Epi-mode tomographic quantitative phase imaging in thick scattering samples", BIOMEDICAL OPTICS EXPRESS, vol. 10, no. 7, 26 June 2019 (2019-06-26), United States, pages 3605, XP093145453, ISSN: 2156-7085, Retrieved from the Internet <URL:https://www.osapublishing.org/viewmedia.cfm?URI=boe-10-7-3605> DOI: 10.1364/BOE.10.003605
- ROBLES FRANCISCO E: "Epi-mode tomographic quantitative phase imaging in thick scattering samples", PROGRESS IN BIOMEDICAL OPTICS AND IMAGING, SPIE - INTERNATIONAL SOCIETY FOR OPTICAL ENGINEERING, BELLINGHAM, WA, US, vol. 11251, 20 February 2020 (2020-02-20), pages 112511Y - 112511Y, XP060129656, ISSN: 1605-7422, ISBN: 978-1-5106-0027-0, DOI: 10.1117/12.2547693
- HU JUNBAO ET AL: "Higher Order Transport of Intensity Equation Methods: Comparisons and Their Hybrid Application for Noise Adaptive Phase Imaging", IEEE PHOTONICS JOURNAL, IEEE, USA, vol. 11, no. 3, June 2019 (2019-06-01), pages 1 - 14, XP011729318, DOI: 10.1109/JPHOT.2019.2919543
- BON PIERRE ET AL: "Self-interference 3D super-resolution microscopy for deep tissue investigations", NATURE METHODS, NATURE PUB. GROUP, NEW YORK, vol. 15, no. 6, 30 April 2018 (2018-04-30), pages 449 - 454, XP036518043, ISSN: 1548-7091, [retrieved on 20180430], DOI: 10.1038/S41592-018-0005-3
- VELLEKOOP IVO M ET AL: "Digital optical phase conjugation of fluorescence in turbid tissue", APPLIED PHYSICS LETTERS, A I P PUBLISHING LLC, US, vol. 101, no. 8, 20 August 2012 (2012-08-20), pages 81108 - 81108, XP012164782, ISSN: 0003-6951, [retrieved on 20120822], DOI: 10.1063/1.4745775
- BON P ET AL: "Quadriwave lateral shearing interferometry for quantitative phase microscopy of living cells", OPTICS EXPRESS, OSA PUBLISHING, US, vol. 17, no. 15, 20 July 2009 (2009-07-20), pages 13080 - 13094, XP002581601, ISSN: 1094-4087, [retrieved on 20090716]
- WOLFGANG OSTEN ET AL: "Recent advances in digital holography [Invited]", APPLIED OPTICS, vol. 53, no. 27, 30 July 2014 (2014-07-30), pages G44, XP055140304, ISSN: 0003-6935, DOI: 10.1364/AO.53.000G44

## Description

La présente invention concerne la caractérisation par imagerie de tissus biologiques, en particulier de micro-tissus biologiques.

En recherche comme en thérapie, il est essentiel de pouvoir caractériser les cellules et tissus biologiques vivants, en particulier pendant ou après une culture cellulaire, notamment pour contrôler la prolifération cellulaire et/ou la qualité des cellules et du tissu et/ou pour suivre la différenciation des cellules et/ou pour suivre l'organisation du tissu et/ou pour déterminer le ou les phénotypes des cellules constituant un tissu, etc.

Toutefois, les techniques d'imagerie actuelles, qui sont en particulier celles mettant en œuvre la microscopie à fluorescence, l'histologie, la mesure de capacitance, la densité optique et l'imagerie à transmission standard, ne le permettent pas.

La microscopie à fluorescence est utilisée couplée avec des sondes fluorescentes telles que des anticorps ou de la fluorescence endogène en modifiant génétiquement les cellules. Plusieurs techniques mettant en œuvre la microscopie à fluorescence sont généralement utilisées, en particulier la microscopie confocale, la microscopie à nappe de lumière (ou SPIM pour « Selective Plane Illumination Microscopy »), la microscopie multiphotonique, la cytométrie de flux (« facs »). Ces techniques sont très établies mais elles requièrent une fixation (mort des cellules) et/ou des conditions limitantes (marquage uniquement de protéines extracellulaires) ou non compatibles avec de la culture cellulaire dans un objectif de thérapie cellulaire, telles que l'ajout de produits non GMP destructifs ou qui entraînent la modification génétique des cellules. Elles ne sont donc pas adaptées à la caractérisation de tissus vivants car elles sont invasives, souvent destructrices et très lentes.

Les techniques d'histologie consistent à fixer puis marquer les tissus. Elles entraînent là encore la destruction des cellules et présentent les mêmes inconvénients que la microscopie à fluorescence.

La mesure de biomasse par sonde à capacitance part de l'a priori que les cellules vivantes peuvent être considérées comme des condensateurs. Cette mesure ne prend donc en compte que la surface extérieure accessible de cellules ayant une membrane intègre. Le cas des agrégats de cellules et des micro-tissus est plus complexe et dépendra de l'étanchéité des connexions entre cellules. Contrairement aux précédentes méthodes, celle-ci est non invasive mais elle ne permet d'avoir des informations que sur le seul volume non accessible ou sur la surface de ce volume, ce qui est trop limitatif et imprécis pour la caractérisation de tissus.

Les techniques d'imagerie à transmission standard, comme par exemple le contraste de phase quantitatif sont rapides et non invasives. La mesure de phase en imagerie est une mesure du retard local d'un faisceau lumineux après interaction avec l'objet étudié. Les dispositifs utilisés pour l'imagerie de phase sont basés sur le phénomène d'interférences optiques pour encoder en information d'intensité lumineuse l'information de phase. Différentes techniques en imagerie de phase pour la microscopie sont décrites notamment dans (Park, Y., Depeursinge, C. & Popescu, G. Quantitative phase imaging in biomedicine. Nature Photon 12, 578-589 (2018) doi:10.1038/s41566-018-0253-x). L'imagerie de phase est aujourd'hui utilisée pour la caractérisation d'échantillons fins (cellules isolées ou des coupes de micro-tissus d'épaisseurs inférieures à 10µm) mais n'est pas utilisable pour des micro-tissus ou tissus plus grands. En effet les techniques actuellement utilisées en imagerie de phase ne permettent pas une mesure quantitative de la phase dans un tissu. Elles restent qualitatives et non quantitatives et sont par conséquent difficilement utilisables pour la caractérisation d'objets épais.

Le document Robles et al "Epi-mode tomographic quantitative phase imaging in thick scattering samples", PROGRESS IN BIOMEDICAL OPTICS AND IMAGING, SPIE - INTERNATIONAL SOCIETY FOR OPTICAL ENGINEERING BELLINGHAM, WA, US, vol 11251, 20 février 2020, présente une technique d'imagerie de phase quantitative pour des échantillons épais. Bien qu'elle permette l'imagerie en profondeur, cette méthode n'est pas optimisée pour la caractérisation rapide et non invasive de micro-tissus vivants en culture.

L'article de Hu Junbao et al. "Higher Order Transport of Intensity Equation Methods", IEEE PHOTONICS JOURNAL, IEEE, USA, vol 11, no. 3, juin 2019, décrit des méthodes avancées d'équation de transport d'intensité pour l'imagerie de phase. Cependant, ces techniques nécessitent l'acquisition de multiples images, limitant leur utilisation pour la caractérisation rapide de tissus vivants en culture.

Le document Bon Pierre et al., "Self-interference 3D super-resolution microscopy for deep tissue investigations", NATURE METHODS, NATURE PUB. GROUP, NEW YORK, vol. 15, n°6, 30 avril 2018, présente une technique de microscopie super-résolution 3D pour l'imagerie en profondeur. Malgré sa haute résolution, cette méthode est complexe et peu adaptée à la caractérisation rapide et non invasive de micro-tissus biologiques en culture.

L'article Bon Pierre et al. "Quadriwave lateral shearing interferometry for quantitative phase microscopy of living cells", OPTICS EXPRESS, OSA PUBLISHING, US, vol. 17, n°15, 20 juillet 2009, décrit une technique d'interférométrie pour l'imagerie de phase quantitative. Cette approche, bien qu'applicable aux cellules vivantes, est limitée aux échantillons fins et n'est pas adaptée aux micro-tissus épais en culture.

La demande US 2018/113064 A1 décrit une méthode pour déterminer l'état d'une cellule. Bien que cette approche permette une analyse cellulaire, elle n'est pas optimisée pour la caractérisation rapide et complète de microtissus biologiques entiers en culture.

Le brevet US 2014/375792 A1 présente des systèmes et méthodes de microscopie de phase quantitative auto-référencée. Cette technique nécessite un alignement précis des composants optiques et n'est pas spécifiquement conçue pour l'analyse rapide et non invasive de micro-tissus vivants en culture.

Le document US 2020/096941 A1 décrit un appareil de mesure pour l'analyse de cellules en culture. Cette méthode n'offre pas une caractérisation complète et rapide des micro-tissus biologiques vivants au cours de leur culture.

L'article WOLFGANG OSTEN ET AL "Recent advances in digital holography", APPLIED OPTICS, vol. 53, no. 27, 30 juillet 2014, présente une vue d'ensemble des progrès récents en holographie numérique. Bien que ces techniques offrent des avantages pour l'imagerie de phase, elles ne sont pas spécifiquement adaptées à la caractérisation rapide, complète et non invasive de micro-tissus biologiques vivants en culture.

Enfin, la mesure de densité optique, obtenue par des mesures de la phase et permettant un accès à la masse de l'échantillon, ne permet pas de caractériser des tissus de taille supérieure à 10µm. L'objectif de l'invention est de pallier ces différentes problématiques de l'art antérieur, et de proposer une solution pour un procédé de caractérisation in vitro de tissus vivants, complet, rapide et non invasif, qui permette de caractériser en particulier des micro-tissus biologiques vivants, notamment au cours ou à la fin d'une culture cellulaire, en recherche ou en thérapie.

### Résumé de l'invention

L'invention est définie dans la revendication 1.

Selon l'invention, la mesure de la phase telle que réalisée actuellement sur les cellules et micro tissus très fins utilise obligatoirement un faisceau de référence ce qui conduit à une mesure absolue non adaptée car elle ne permet pas de mesurer la masse et de quantifier un certain nombre de paramètres.

C'est pourquoi, pour répondre à l'objectif de l'invention, les inventeurs ont mis au point un procédé de caractérisation in vitro d'un micro-tissu biologique humain, animal ou végétal dont la plus petite dimension est supérieure ou égale à 20µm, ledit procédé consistant à utiliser une technique de mesure de la phase sans faisceau de référence et ne nécessitant pas d'avoir recours à un marquage fluorescent.

En effet, selon l'invention, seules les techniques de mesure de la phase sans faisceau de référence, c'est-à-dire les techniques de mesures indirectes ou encore appelées mesures de la phase relative, peuvent fonctionner pour caractériser des micro-tissus dont la plus petite dimension est supérieure ou égale à 20µm. De plus, selon l'invention il est important de gérer la cohérence du faisceau utilisé pour l'éclairage. En particulier il convient que la cohérence au niveau de l'échantillon soit telle que la tavelure (« *speckle »)* générée par l'échantillon (micro-tissu) soit réduite, préférentiellement le contraste de la tavelure est inférieur à 75% d'un contraste maximale unitaire, encore plus préférentiellement 50%, idéalement 10%. En pratique cette réduction de la tavelure se fait en diminuant la cohérence spatiale et/ou la cohérence temporelle. La cohérence spatiale de l'éclairage doit être telle que l'ouverture numérique d'illumination ne dépasse pas 90%, préférentiellement 50%, idéalement 25% de l'ouverture numérique du système d'imagerie, de façon à mesurer les paramètres de l'échantillon de façon indépendante de la cohérence du faisceau.

Avantageusement, l'utilisation d'une technique de mesure de la phase sans faisceau de référence permet de caractériser de façon quantitative à haut débit des micro-tissus biologiques vivants sans les détruire, ni les modifier.

Ceci permet en particulier de :
- mesurer l'augmentation de la biomasse d'un micro-tissu pendant sa culture et/ou son amplification,
- déterminer la viabilité des cellules,
- contrôler la qualité d'un micro-tissu,
- suivre la différenciation et/ou l'organisation d'un micro-tissu durant sa maturation,
- déterminer le phénotype de cellules du micro-tissu, et/ou
- confirmer l'absence de cellules non différenciées dans le micro-tissu.

L'invention a donc également pour objet l'utilisation du procédé de caractérisation pour ces applications en particulier.

### Brève description des Figures

- la Figure 1 est une image de capsules en hydrogel contenant des cellules humaines induites à la pluripotence, obtenue par imagerie par mesure de la phase sans faisceau de référence, selon le protocole décrit dans l'exemple.
- la Figure 2 est une image de capsules en hydrogel contenant des cellules humaines induites à la pluripotence, obtenue par imagerie par mesure de l'intensité de la phase (mesure absolue), selon le protocole décrit dans l'exemple.
- la Figure 3 est une représentation schématique de la mise en œuvre d'une variante du procédé en ligne pour caractériser des micro-tissus biologiques contenus dans un bioréacteur.
- la Figure 4 est une représentation schématique du système d'imagerie de mesure de la phase sans faisceau de référence utilisé pour le procédé de caractérisation selon l'invention décrit dans l'exemple 1.
- La Figure 5a représente des images prises selon le procédé selon l'invention de micro-tissus issus de l'encapsulation de cellules souches humaines induites (Gibco Human Episomal) dans des capsules d'alginate après 6 jours de culture dans un milieu de croissance (MTesr1 supplémenté) selon le protocole de l'exemple 2. Le micro-tissu est composé de cellules souches formant un cyste unique et répondant à des critères d'homogénéité de distribution de cellules et de rondeur permettant de les classer dans la catégorie des micro-tissus acceptables.
- La Figure 5b représente des images prises selon le procédé selon l'invention de micro-tissus issus de l'encapsulation de cellules souches humaines induites (Gibco Human Episomal) dans des capsules d'alginate après 6 jours de culture dans un milieu de croissance (MTesr1 supplémenté) selon le protocole de l'exemple 2. Le micro-tissu est composé de cellules souches formant plusieurs cystes et/ou présentant une inhomogénéité de distribution de cellules et de rondeur du permettant de les classer dans la catégorie des micro-tissus non-acceptables.
- La Figure 5c (gauche) représente une image prise selon le procédé selon l'invention de micro-tissus issus de l'encapsulation de cellules souches humaines induites (Gibco Human Episomal) dans des capsules d'alginate après 6 jours de culture dans un milieu de croissance (MTesr1 supplémenté). La Figure 5c (droite) représente le même objet acquis à l'aide de microscopie multiphotonique. Les noyaux des cellules sont marqués par 10 ug/mL Hoechst 33342 (Thermofisher). Environ la moitié des cellules sont visibles. Le nombre total des cellules est de 500 environ.
- la Figure 6 est une représentation graphique des résultats comparatifs de mesure de masse et de surface entre des micro-tissus de cellules souches dans des capsules d'alginate à différents temps (1 à 6 jours) après encapsulation montrant la croissance quadratique de la masse et la diversité de masse et de maturité pour des échantillons produits au même moment selon l'exemple 2,
- la Figure 7 est une représentation graphique des résultats comparatifs de mesure de masse normalisé à la surface entre des capsules contenant des micro-tissus et des capsules vides selon l'exemple 2.

### Définitions

Par « absorption locale » du micro-tissu au sens de l'invention, on entend l'atténuation de la lumière due à une perte de photon local par absorption de la lumière et non diffusion.

Par « alginate » au sens de l'invention des polysaccharides linéaires formés à partir de β-D-mannuronate et α-L-guluronate, des sels et des dérivés de ceux-ci.

Par « capsule en hydrogel » au sens de l'invention on entend une structure tridimensionnelle formée à partir d'une matrice de chaînes polymères gonflée par un liquide et préférentiellement de l'eau.

Par « cellules humaines » au sens de l'invention on entend des cellules humaines ou des cellules de mammifères non humains immunologiquement humanisées. Même lorsque cela n'est pas précisé, les cellules, les cellules souches, les cellules progénitrices et les tissus selon l'invention, sont constitués ou sont obtenus à partir de cellules humaines ou à partir de cellules de mammifères non humains immunologiquement humanisées.

Par « cellule progénitrice » au sens de l'invention, on entend une cellule souche déjà engagée dans la différenciation cellulaire (par exemple en cellules de la rétine) mais pas encore différenciée. Une cellule progénitrice est une cellule qui a tendance à se différencier en un type spécifique de cellule. Elle est donc déjà plus spécifique qu'une cellule souche. Les cellules progénitrices ne peuvent se diviser qu'un nombre limité de fois, étant naturellement sujette à l'érosion de leurs télomères.

Par « cellule souche embryonnaire » au sens de l'invention on entend une cellule souche pluripotente de cellule dérivée de la masse cellulaire interne du blastocyste. La pluripotence des cellules souches embryonnaires peut être évaluée par la présence de marqueurs tels que les facteurs de transcription OCT4 et NANOG et des marqueurs de surface comme SSEA3/4, Tra-1-60 et Tra-1-81. Les cellules souches embryonnaires peuvent être obtenues sans destruction de l'embryon dont elles sont issues, par exemple à l'aide de la technique décrite dans Chang et al. (Cell Stem Cell, 2008, 2(2)) : 113-117). Eventuellement les cellules souches embryonnaires d'êtres humains peuvent être exclues.

Par « cellule souche pluripotente » ou « cellule pluripotente » au sens de l'invention, on entend une cellule qui a la capacité de former tous les tissus présents dans l'organisme d'origine entier, sans pour autant pouvoir former un organisme entier en tant que tel. Il peut s'agir en particulier de cellules souches pluripotentes induites, de cellules souches embryonnaires ou de cellules MUSE (pour « Multilineage-differentiating Stress Enduring »). Les cellules souches pluripotentes maintiennent la longueur de leurs télomères et peuvent conservent la capacité de se diviser sans limite claire de nombre de cycle cellulaire à l'opposé des cellules progénitrices.

Par « cellule souche pluripotente induite » au sens de l'invention on entend une cellule souche pluripotente induite à la pluripotence par reprogrammation génétique de cellules somatiques différenciées. Ces cellules sont notamment positives pour les marqueurs de pluripotence, comme la coloration à la phosphatase alcaline et l'expression des protéines NANOG, SOX2, OCT4 et SSEA3/4. Des exemples de procédés permettant l'obtention de cellules souches pluripotentes induites sont décrits dans les articles Yu et al. (Science 2007, 318 (5858) : 1917-1920), Takahashi et al (Cell, 207, 131(5) : 861-872) et Nakagawa et al (Nat Biotechnol, 2008, 26(1) : 101-106).

Par cellules « différenciées » au sens de l'invention on entend des cellules qui présentent un phénotype particulier, par opposition à des cellules souches pluripotentes qui ne sont pas différenciées.

Par « cohérence » du faisceau au sens de l'invention, on entend la cohérence spatio-temporelle à savoir l'étendue spatiale et la largeur spectrale de la source d'illumination.

Par « densité » du micro-tissu au sens de l'invention, on entend la masse d'une unité de volume divisée par la masse du même volume de milieu de culture.

Par « micro-tissu » ou « micro-tissu biologique » au sens de l'invention, on entend un tissu biologique ou un échantillon de tissu biologique dont la plus grande dimension est inférieure ou égale à 1 cm.

Par « phase », au sens de l'invention on entend le retard de front d'onde lumineux, déphasage relatif et différence de chemin optique entre l'environnement du micro-tissu et le niveau de base du milieu dans lequel il est plongé.

Par « technique de mesure de la phase » au sens de l'invention, on entend toute technique capable de mesurer de façon quantitative la phase de la lumière.

Par « technique de mesure de la phase sans faisceau de référence » au sens de l'invention, on entend les techniques capables de remonter à la composante de phase de la lumière sans avoir recours à l'utilisation d'un faisceau externe, dit « de référence », n'ayant pas interagi avec le micro-tissu.

Par « tissu » ou « tissu biologique »au sens de l'invention, on entend le sens commun de tissu en biologie c'est-à-dire le niveau d'organisation intermédiaire entre la cellule et l'organe. Un tissu est un ensemble de cellules semblables et de même origine (le plus souvent issus d'un lignage cellulaire commun, bien qu'elles puissent trouver leur origine par association de lignages cellulaires distincts)., regroupées en amas, réseau ou faisceau (fibre). Un tissu forme un ensemble fonctionnel, c'est-à-dire que ses cellules concourent à une même fonction. Les tissus biologiques se régénèrent régulièrement et sont assemblés entre eux pour former des organes. Un tissu peut comprendre des cellules différenciées et des cellules souches. Typiquement les cellules souches pluripotentes forment un tissu de type epithélial, qualifié d'épiblastique (citation : Self-organization of the human embryo in the absence of maternal tissues, Shahbazi et al., Nat Cell Biol. 2016, doi: 10.1038/ncb3347) .

Par « texture » d'un micro-tissu au sens de l'invention, on entend la rugosité locale de l'image et son contenu fréquentielle local.

### Description détaillée

L'invention a donc pour objet un procédé de caractérisation in vitro d'un micro-tissu biologique d'eucaryote, notamment un micro-tissu humain, animal ou végétal, dont la plus petite dimension est supérieure ou égale à 20µm, encore plus préférentiellement supérieure ou égale à 30 µm. Le procédé consiste à caractériser le micro-tissu biologique dans son intégralité et pas seulement une partie du micro-tissu.

Le micro-tissu biologique est préférentiellement un micro-tissu dont la plus grande dimension est inférieure ou égale à 10 mm, encore plus préférentiellement inférieure ou égale à 1mm et en particulier inférieure ou égale à 500µm, notamment inférieure ou égale à 200 µm.

Le micro-tissu biologique peut être un micro-tissu comprenant des cellules eucaryotes, en particulier des cellules humaines, ou des cellules animales (non humaines) notamment des cellules d'amniotes et en particulier des cellules de mammifères, ou des cellules végétales.

Le micro-tissu biologique lorsqu'il s'agit d'un micro-tissu humain ou animal peut par exemple être choisi parmi les micro-tissus épithéliaux, conjonctifs, musculaires ou nerveux.

Selon un mode de réalisation, le micro-tissu peut notamment comprendre :
- des cellules différenciées cardiaques ou des cellules rétiniennes ou des cellules neurales ou des cellules du foie ou des chondrocytes ou des kératinocytes ou des cellules lymphoïdes ou des cellules souches hématopoiétiques ou des cellules souches mésenchymateuses, et/ou :
- des cellules souches progénitrices
- des cellules endothéliales.

Selon un autre mode de réalisation le micro-tissu peut comprendre ou être constitué de cellules pluripotentes sous la forme d'un épiblaste.

Le micro-tissu biologique lorsqu'il s'agit d'un micro-tissu humain ou animal par exemple être choisi parmi les différentes phases du développement embryonnaire ou fœtal, en particulier dans les phases précoces du développement dans le cadre de la fécondation in vitro à but reproductif (chez l'Homme ou l'animal) ou de recherche (chez l'Homme ou l'animal) ou de production animale.

Le micro-tissu biologique lorsqu'il s'agit d'un micro-tissu végétal peut par exemple être choisi parmi les méristèmes, les parenchymes, les tissus conducteurs, les tissus de soutien, les tissus de revêtement ou de protection, les tissus de sécrétion et les tissus nourriciers.

Le micro-tissu peut être entouré au moins partiellement d'une matrice extracellulaire. La couche de matrice cellulaire peut être constituée par de la matrice cellulaire sécrétée par des cellules du micro-tissu et/ou par de la matrice extracellulaire ajoutée. La couche de matrice extracellulaire peut former un gel. Elle comprend préférentiellement un mélange de protéines et de composés extracellulaires nécessaires à la culture des cellules constituant le micro-tissu. Préférentiellement, la matrice extracellulaire comprend des protéines structurelles, telles que du collagène, des laminines, de l'entactine, de la vitronectine, ainsi que des facteurs de croissance, tels que du TGF-béta et/ou de l'EGF. La couche de matrice extracellulaire peut consister en ou comprendre du Matrigel^{®} et/ou de la Geltrex^{®} et/ou une matrice type hydrogel d'origine végétale comme des alginates modifiés ou d'origine synthétique ou de copolymère de poly(N-isopropylacrylamide) et de poly(ethylene glycol) (PNIPAAm-PEG) type Mebiol^{®}.

Selon une variante le micro-tissu peut-être un micro-tissu encapsulé dans un microcompartiment ou une capsule comprenant une couche externe en hydrogel, tels que par exemple les microcompartiments décrits dans la demande de brevet WO2018/096277 A1.

On parle de capsule en hydrogel. Préférentiellement l'hydrogel utilisé est biocompatible, c'est-à-dire qu'il n'est pas toxique pour les cellules. La capsule d'hydrogel doit permettre la diffusion d'oxygène et de nutriment pour alimenter les cellules contenues dans le microcompartiment et permettre leur survie. La couche externe en hydrogel peut être une couche externe comprenant de l'alginate. Elle peut être constituée exclusivement d'alginate. L'alginate peut être en particulier un alginate de sodium, composé à 80% d'α-L-guluronate et 20% de β-D-mannuronate, avec une masse moléculaire moyenne de 100 à 400 kDa et une concentration totale comprise entre 0,5 et 5% en masse. La capsule d'hydrogel permet notamment de protéger les cellules du milieu extérieur et de limiter la prolifération incontrôlée des cellules.

Le micro-tissu peut se présenter sous toute forme en trois dimensions, c'est-à-dire qu'il peut avoir la forme de tout objet de l'espace. Il peut se présenter par exemple sous la forme d'un ovoïde creux ou plein, d'un cylindre creux ou plein, d'un tuboïde ou d'un tube creux ou plein, d'un sphéroïde ou d'une sphère creuse ou pleine, de monocouches partiellement repliées sur elles-mêmes (2,5D). C'est la couche externe du micro-tissu ou la couche de matrice extracellulaire lorsqu'elle est présente, qui confère sa taille et sa forme au micro-tissu. Un exemple de micro-tissu plein est le sphéroïde cardiaque utilisé en bioproduction (https://doi.org/10.1016/j.bbamcr.2015.11.036)

Selon un mode de réalisation de l'invention, le micro-tissu peut être un micro-tissu biologique humain ou animal destiné à être greffé chez l'homme ou l'animal.

Le micro-tissu lors de la mise en œuvre du procédé peut être réalisé sur un micro-tissu vivant congelé ou non congelé.

Le procédé selon l'invention comprend la caractérisation du micro-tissu en imagerie par une technique de mesure de la phase sans faisceau de référence.

II est important de gérer la cohérence du faisceau utilisé pour l'éclairage. En particulier il convient que la cohérence au niveau du micro-tissu soit telle que la tavelure *(« speckle* ») générée par l'échantillon (micro-tissu) soit réduite, préférentiellement le contraste de la tavelure est inférieur à 75% du contraste unitaire, encore plus préférentiellement 50%, idéalement 10%. En pratique cette réduction de la tavelure se fait en diminuant la cohérence spatiale et/ou la cohérence temporelle. La cohérence spatiale de l'éclairage doit être telle que l'ouverture numérique d'illumination ne dépasse pas 90%, préférentiellement 75%, idéalement 50% de l'ouverture numérique du système d'imagerie, de façon à mesurer les paramètres de l'échantillon (micro-tissu) de façon indépendante de la cohérence du faisceau.

Selon l'invention, le procédé est réalisé avec un faisceau semi-cohérent spatialement c'est-à-dire que :
- la largueur spectrale de la source, c'est-à-dire l'étendue spectrale de l'illumination appelée encore la longueur d'onde d'illumination (correspond à la cohérence temporelle d'illumination) est d'au minimum 5 nm dans le visible et au maximum 600 nm; idéalement environ 100 nm, par exemple 100 nm ; et
- l'ouverture numérique d'illumination (correspondant à la cohérence spatiale d'illumination) est d'au minimum 5% et au maximum 90% de l'ouverture numérique du système d'imagerie, idéalement environ 50%, par exemple 50%.

Cette caractéristique permet en particulier de garantir une mesure quantitative de la phase comparable quel que soit l'échantillon (micro-tissu) et les paramètres d'illumination tout en mesurant les paramètres du micro-tissu dans son intégralité.

Préférentiellement la technique de mesure de la phase sans faisceau de référence est choisie parmi :
- l'analyse de front d'onde
- la modulation dynamique de la phase ou de l'intensité lumineuse dans la pupille du système d'illumination ou d'imagerie
- l'imagerie multiple d'intensité lumineuse avec modification du plan de mise au point.

Lorsque la technique de mesure de la phase sans faisceau de référence est l'analyse de front d'onde, elle est préférentiellement réalisée à l'aide de l'imagerie des gradients du front d'onde, et en particulier une technique de l'imagerie des gradients du front d'onde choisie parmi :
- la méthode Shack-Hartmann (Gong, H. et al. Optical path difference microscopy with a Shack Hartmann wavefront sensor. Opt. Lett. (2017) oi:10.1364/OL.42.002122),
- la méthode Hartmann modifiée (ou non) (Bon, P., Maucort, G., Wattellier, B. & Monneret, S. Quadriwave lateral shearing interferometry for quantitative phase microscopy of living cells. Opt. Express 17, 13080-13094 (2009)),
- le partitionnement de la pupille (Parthasarathy, A. B., Chu, K. K., Ford, T. N. & Mertz, J. Quantitative phase imaging using a partitioned detection aperture. Opt. Lett. 37, 4062-4064 (2012)),
- l'imagerie de champ de speckle (Berto, P., Rigneault, H. & Guillon, M. Wavefront sensing with a thin diffuser. Opt. Lett. 42, 5117-5120 (2017)).

Préférentiellement, la technique de mesure de la phase sans faisceau de référence utilisée dans le procédé selon l'invention est la méthode de Hartmann modifiée car c'est la technique permettant d'obtenir le meilleur compromis en termes de stabilité, sensibilité et compacité pour la caractérisation de micro-tissus.

Lorsque la technique de mesure de la phase sans faisceau de référence est la modulation dynamique de la phase ou de l'intensité lumineuse dans la pupille du système d'illumination ou d'imagerie, elle est préférentiellement réalisée à l'aide de :
- ptychographie (Zheng, G., Horstmeyer, R. & Yang, C. Wide-field, high-resolution Fourier ptychographic microscopy. Nat. Photonics 7, 739 (2013)), ou
- la modulation de phase sélective de certaines fréquences dans la pupille (Wang, Z. et al. Spatial light interference microscopy (SLIM). Opt. Express 19, 1016-1026 (2011)).

Préférentiellement, la technique de mesure de la phase sans faisceau de référence utilisée dans le procédé selon l'invention est la technique de ptychographie car la quantification de la phase y est plus directe que la modulation de phase sélective qui ne donne des images que peu quantitatives.

Lorsque la technique de mesure de la phase sans faisceau de référence est l'imagerie multiple d'intensité lumineuse avec modification du plan de mise au point, elle est préférentiellement réalisée à l'aide de :
- l'imagerie multiplan simultanée (Descloux, A. et al. Combined multi-plane phase retrieval and super-resolution optical fluctuation imaging for 4D cell microscopy. Nat. Photonics 12, 165-172 (2018)) ou l'imagerie multiplan séquentiel (Soto, J. M., Rodrigo, J. A. & Alieva, T. Label-free quantitative 3D tomographic imaging for partially coherent light microscopy. Opt. Express 25, 15699-15712 (2017) ou Barty, A., Nugent, K. A., Paganin, D. & Roberts, A. Quantitative optical phase microscopy. Opt. Lett. 23, 817-819 (1998)).

Préférentiellement, la technique de mesure de la phase sans faisceau de référence utilisée dans le procédé selon l'invention est la technique simultanée, car elle est rapide, même si elle présente une complexité plus importante que l'imagerie multiplan séquentiel.

Quelle que soit la technique de mesure de la phase sans faisceau de référence, le procédé comprend préférentiellement la mesure de la phase et éventuellement de l'intensité lumineuse de la lumière ayant traversé le micro-tissu.

Préférentiellement, le procédé selon l'invention est un procédé de caractérisation in vitro d'un micro-tissu biologique d'eucaryote en imagerie par une technique de mesure de la phase sans faisceau de référence, ledit procédé comprenant au moins l'étude de l'organisation des cellules dans le micro-tissu, préférentiellement au moins la topologie du micro-tissu et/ou le positionnement relatif des cellules dans le micro-tissu.

Selon un mode de réalisation préféré, le procédé comprend la mesure :
- de la densité du micro-tissu, à partir de la mesure de la phase, et
- éventuellement de l'absorption locale du micro-tissu, à partir de la mesure de la phase et de la mesure de l'intensité lumineuse de la lumière ayant traversé le micro-tissu.

La densité du micro-tissu peut être mesurée à partir de la mesure de la phase comme suit :
1) la zone de l'image contenant le micro-tissu (appelée zone utile) est séparée du reste (appelé fond, généralement le milieu de culture) ;
2) la valeur de la phase du fond est soustraite à la phase de la zone utile;
3) après cette soustraction, la phase est convertie si besoin en différence de chemin optique (exprimé en unité métrique) et sommée sur l'intégralité de la zone utile ;
4) cette valeur est multipliée par la surface d'un pixel élémentaire de l'image, ramené dans le plan objet : on obtient une valeur en unité métrique au cube ;
5) cette grandeur est divisée par l'incrément de réfraction spécifique (Barer, Interference microscopy and mass determination, Nature, 1952) qui est de 0.18 µm³/pg en moyenne et qui peut être ajustée pour chaque tissu : on obtient ainsi une mesure de la masse dite sèche (masse totale - masse du milieu de culture) intégrée surtout l'échantillon (micro-tissu).

Un comparatif et descriptif de cette technique est disponible (Zangle, T. et Teitell, M.A., Live-cell mass profiling: on emerging approach in quantitative biophysics, Nature Methods, 2014)

La densité est exprimée en unité massique (g).

L'absorption locale du micro-tissu peut être mesurée à partir de la mesure de la phase et de la mesure de l'intensité lumineuse de la lumière ayant traversé le micro-tissu comme suit. Par une mesure conjointe la phase *φ* et de l'intensité *I*, on obtient le champ électromagnétique *E = Ie^{iφ}.* Cette grandeur complexe peut être décomposée en extrayant la partie réelle et imaginaire dans l'espace de Fourier numérique du champ électromagnétique (via une transformée de Fourier). En revenant dans l'espace direct (via une transformée de Fourier inverse) de la composante réelle de l'espace de Fourier on peut remonter à la composante de l'absorption locale. La mesure de l'absorption est exprimée en photon/cm².

Préférentiellement, le procédé selon l'invention comprend la mesure d'au moins un des paramètres suivants :
- dimensions du micro-tissu
- dimensions d'au moins une des cellules du micro-tissu
- nombre de cellules dans le micro-tissu
- masse globale et locale du micro-tissu
- densité globale et locale du micro-tissu
- distribution de masse dans le micro-tissu
- organisation des cellules dans le micro-tissu : topologie du micro-tissu et/ou positionnement relatif des cellules dans le micro-tissu
- viabilité des cellules du micro-tissu
- texture.

Les dimensions du micro-tissu peuvent être mesurées à partir de la mesure de la phase comme suit. Les dimensions dans le plan de l'image sont extraites par détourage automatique (algorithme de détection de bord de type Otsu par exemple, ou détourage manuel) et les dimensions sont obtenues par un ajustement du détourage par une ellipse (dans le cas d'un micro tissu ovoïde).

Les dimensions sont exprimées en micromètre.

Les dimensions d'une ou plusieurs cellule(s) du micro-tissu peuvent être mesurées à partir de la mesure de la phase comme suit. Lorsque la résolution optique est meilleure que la taille d'une cellule, on procède à un détourage manuel ou automatique au sein du micro-tissu (algorithme de détection de bord ou ligne de partage des eaux). Les dimensions sont ensuite obtenues par un ajustement de chaque détourage automatique par une ellipse.

Les dimensions d'une cellule sont exprimées en micromètre.

La masse globale du micro-tissu peut être mesurée à partir de la mesure de la phase comme suit. La somme de l'information de phase (au sens du chemin optique, exprimé en µm) sur la zone contenant le micro-tissu (obtenu par détourage automatique ou manuel) est ensuite multipliée par la surface d'un pixel de phase ramené dans l'espace de l'objet (exprimée en µm²) puis multipliée par l'incrément spécifique (généralement 0.18pg/µm3) pour obtenir la mesure de masse globale.

La masse globale est exprimée en microgramme.

La masse locale du micro-tissu peut être mesurée à partir de la mesure de la phase comme suit. Le même procédé que pour le point précédent est appliqué mais en sommant la phase uniquement sur une partie choisie du micro-tissu.

La masse locale est exprimée en microgramme.

La densité globale du micro-tissu peut être mesurée à partir de la mesure de la phase comme suit. La masse est mesurée à partir de la phase. Les dimensions transverses dans le plan image sont obtenues avec l'image de phase. La dimension dans le plan orthogonal de l'image de phase (appelé épaisseur) est obtenue : a) soit pas une reconstruction 3D de l'objet dans différents plans d'imagerie, b) soit d'une hypothèse sur la forme de l'objet (en général ovoïde), c) soit d'une hypothèse sur l'indice de réfraction optique moyen du micro-tissu et du milieu qui permet, en divisant la phase (au sens de la différence de chemin optique) par la différence d'indice de réfraction, de remonter à l'épaisseur du micro-tissu. Les trois dimensions sont combinées pour obtenir le volume de l'échantillon (micro-tissu). En divisant la masse par le volume, on remonte à la masse volumique.

La densité globale est exprimée en g/cm³

La densité locale du micro-tissu peut être mesurée à partir de la mesure de la phase comme suit. Le même protocole que pour la mesure de la densité globale est utilisé mais en restreignant la zone mesurée à une sous partie du micro-tissu. La densité locale est exprimée en g/cm³.

La distribution de masse dans le micro-tissu peut être mesurée à partir de la mesure de la phase comme suit. Des mesures de masse locale sont effectuées sur des sous-parties du micro-tissu couvrant tout ou une partie du micro-tissu. Une analyse statistique de ses masses (type déviation standard/écart-type, moyenne/médiane) est effectuée.

La distribution de masse est exprimée en gramme.

L'organisation des cellules dans le micro-tissu est à prendre au sens histologique du terme comme appréciée par l'homme de l'art et qualifie la topologie du tissu et le positionnement relatifs des cellules et des éléments de matrice extracellulaire. La viabilité des cellules du micro-tissu peut être mesurée à partir de la mesure de la phase comme suit : des mesures de masse locale sont effectuées sur des sous-partie du micro-tissu couvrant tout ou une partie du micro-tissu. Une analyse statistique de ses masses (type déviation standard/écart-type, moyenne/médiane) est effectuée.

La distribution de masse est alors corrélée à une analyse histologique conventionnelle pour générer un jeu de donnée d'entrainement analysé et annoté. Un apprentissage automatique algorithmique et/ou dirigé (type réseau de neurone par exemple) peut alors être effectué sur ce jeu de donné afin d'automatiser le procédé.

L'organisation des cellules dans le micro-tissu est donc qualifiée par un système expert, humain ou non, basé sur la classification histologique.

Les phénomènes de mort cellulaires provoquent un changement de densité et de taille des cellules détectable en Phase. La viabilité des cellules du micro-tissu peut être mesurée à partir de la mesure de la phase comme suit : des mesures de masse locale sont effectuées sur des sous-partie du micro-tissu couvrant tout ou une partie du micro-tissu. Une analyse statistique de ses masses (type déviation standard/écart-type, moyenne/médiane) est effectuée. La distribution de masse est alors corrélée à des mesures de viabilité usuelles comme le bromure d'éthidium (cellules mortes) et la calcéine (cellules vivantes) identifiant le pourcentage de cellules vivantes pour générer un jeu de donnée d'entrainement analysé et annoté. Un apprentissage automatique algorithmique et/ou dirigé (type réseau de neurone par exemple) peut alors être effectué sur ce jeu de donné afin d'automatiser le procédé.

La viabilité des cellules dans le micro-tissu est donc est exprimée en pourcentage de cellules vivantes sur le total des cellules.

La texture du micro-tissu peut être mesurée à partir de la mesure de la phase comme suit. La mesure des statistiques de variation spatiale de la phase incluant la déviation standard et distribution fréquentielle des structures de l'image au sein de région d'intérêt permet de déterminer des paramètres de texture.

La texture est exprimée en unité de phase et en (unité de phase)/µm.

Selon un mode de réalisation, le procédé selon l'invention peut être réalisé in vitro sur des micro-tissus qui ont été préalablement prélevés sur un être humain, un animal ou un végétal. Le procédé peut permettre par exeloke de caractériser la qualité d'un ilot de Langerhans issus de cadavre (notamment sa viabilité) avant greffe chez un patient diabétique, ou encore de caractériser un embryon pré-implantatoire.

Selon un autre mode de réalisation, le procédé selon l'invention peut être réalisé in vitro sur des micro-tissus comprenant des cellules souches pluripotentes ou progénitrices destinées à être différenciées ou sur des micro-tissus comprenant des cellules en cours de différenciation ou sur des micro-tissus comprenant des cellules différenciées obtenues par culture cellulaire à partir de cellules souches pluripotentes ou progénitrices. Le procédé selon l'invention peut être réalisé in vitro sur des micro-tissus constitués de cellules couches pluripotentes destinées à être différenciées ou sur des micro-tissus constitués de cellules en cours de différenciation ou sur des micro-tissus constitués de cellules différenciées obtenues par culture cellulaire à partir de cellules souches pluripotentes ou progénitrices.

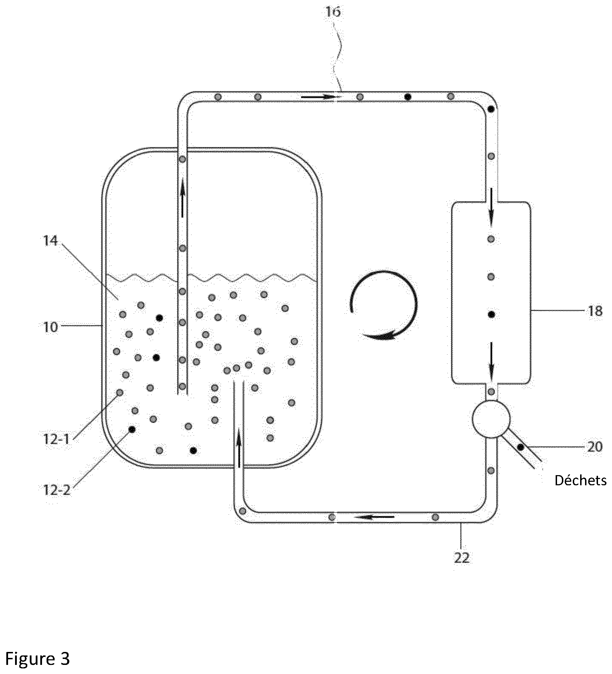

Selon une variante, le procédé selon l'invention est mis en œuvre en ligne sur le contenu d'un bioréacteur. Un exemple d'une telle variante appliqué à la culture cellulaire dans des capsules ou microcompartiments est représenté sur la figure 3. Dans cet exemple, des capsules 12 contenant chacune un micro-tissu sont en suspension dans un milieu de culture 14 dans un bioréacteur 10. Des moyens de sortie 16 disposés sur le bioréacteur permettent de faire sortir les capsules 12 dans leur milieu de culture 14 et de les faire passer dans un système d'imagerie de mesure de la phase sans faisceau de référence 18. En sortie de ce système 18 les capsules comprenant des micro-tissus répondant aux critères de qualité définis par l'utilisateur du bioréacteur, dites capsules normales 12-1 dans leur milieu de culture 14 sont réintégrées via des moyens d'entrée 20 dans le bioréacteur 10 et les capsules indésirables 12-2 ne répondant pas aux critères de qualité définis par l'utilisateur du bioréacteur, sont récupérées via des moyens d'élimination 22 pour être éliminées. Les moyens de sortie 16 peuvent être par exemple une tubulure et une pompe péristaltique Les moyens d'entrée 20 peuvent être par exemple une tubulure. Les moyens d'élimination 22 peuvent être par exemple un système de valve piézo-électrique. Le système 18 peut être tout système d'imagerie adapté à la mesure de la phase sans faisceau de référence tel que l'un de ceux décrits dans la présente demande.

Ceci permet avantageusement de vérifier la qualité des micro-tissus, en particulier en ligne en cours de différenciation ou de maturation. Le procédé selon l'invention, notamment dans le cadre d'une mise en œuvre au cours d'une différenciation ou maturation des cellules formant un micro-tissu dans un bioréacteur, peut ainsi être réalisé :
i) en cellules de flux, c'est-à-dire par recirculation continue du contenu d'une enceinte de culture de type bioréacteur au sein d'un système fluidique stérile à des fins d'analyse et/ou de tri du contenu de la dite enceinte de culture, ou
ii) en prélèvement ponctuel hors du bioréacteur pour analyser à un point particulier dans le temps une partie dudit bioréacteur, typiquement au cours d'un prélèvement à des fins d'analyse et/ou de réensemencement d'un second bioréacteur dans le cadre d'un « seed train » et ou dans le cadre d'une montée en volume et ou de fragmentation du contenu du bioréacteur en plusieurs enceintes ou conditions de contrôle qualité, ou
iii) pour trier les micro-tissus hors ligne au cours de la vidange du bioréacteur à des fins de purification ou de poursuite d'une séquence de production et/ou de différenciation et ou de conditionnement.

Le procédé selon l'invention présente de nombreux avantages par rapport aux méthodes utilisées actuellement. En particulier il peut être mis en œuvre sans détruire ou modifier les micro-tissus étudiés, il est rapide à mettre en œuvre, demande un matériel simple, et permet de mesurer de nombreux paramètres physiques pour caractériser les micro-tissus ce qui n'était pas possible avec les méthodes de l'art antérieur.

Le procédé peut ainsi être utilisé pour de nombreuses applications. Notamment, l'invention a pour objet l'utilisation du procédé pour :
- contrôler la qualité d'un micro-tissu : en effet la mise en œuvre du procédé selon l'invention permet de mesurer des caractéristiques du micro-tissu tels que sa taille, sa densité, le nombre de cellules ou sa texture qui permettent de vérifier la qualité d'un micro-tissu, et/ou
- mesurer l'augmentation de la biomasse d'un micro-tissu pendant sa culture et/ou son amplification : en effet, le procédé selon l'invention permet de mesurer la masse globale ou locale d'un micro-tissu et permet ainsi le suivi de l'augmentation du nombre de cellules dans un micro-tissu au cours de sa culture, sa différenciation et/ou son amplification, et/ou
- suivre la différenciation et/ou l'évolution de la topologie d'un micro-tissu durant sa maturation, et notamment la position relative dans l'espace des cellules le composant : en effet, le procédé selon l'invention permet de mesurer la distribution de masse dans le micro-tissu et/ou l'organisation des cellules dans le micro-tissu et/ou la viabilité des cellules du micro-tissu, en cours de différenciation et/ou de maturation des cellules du micro-tissu, ce qui donne des informations sur la différenciation et/ou la maturation desdites cellules, et/ou
- déterminer le phénotype de cellules du micro-tissu, en effet, le procédé selon l'invention permet de mesurer la masse de chaque cellule et la texture du micro-tissu, ce qui donne des informations sur le phénotype desdites cellules, et/ou
- confirmer l'absence de cellules non différenciées dans le micro-tissu : en effet la mesure de la masse des cellules et/ou la densité des cellules, la texture du micro-tissu et/ou l'organisation des cellules dans le micro-tissu donne(nt) des informations sur la différenciation des cellules du micro-tissu et par conséquent l'éventuelle absence de différenciation desdites cellules.

Selon un mode de réalisation particulier, le micro-tissu peut être un embryon. Ainsi le procédé selon l'invention peut être utilisé pour le screening d'embryon obtenus par fécondation in vitro. La structure histologique d'un embryon bien portant est typique, très reproductible et prédictive du succès de l'implantation de l'embryon chez la mère. En particulier pour décrire cette structure sur embryon appelé à être réimplanté, seules des solutions d'imagerie sans marquage sont envisageables. Pour améliorer le rendement des implantations et réduire le risque d'échec ou, au contraire, d'embryons multiples, les cliniques développent un suivi de plus en plus précis de l'embryon fécondé en préimplantation avec notamment un suivi vidéo du développement. Le procédé selon l'invention permet d'exclure les embryons avec une structure anormale de manière plus efficace en ajoutant une source d'information pertinente et sans marquage, donc non destructive.

Selon un autre mode de réalisation le micro-tissu est un micro-tissu produit à des fins de bioproduction de médicament ou de bioproduction alimentaire.

L'invention est à présent illustrée par un exemple de mise en œuvre du procédé selon l'invention comparé à un exemple de procédé de caractérisation de l'art antérieur.

### Exemples

### Exemple 1

Dans cet exemple, le procédé porte sur l'analyse d'un micro-tissu humain contenu dans un microcompartiment, tel que décrit à l'exemple 1 de la demande WO2018/096277 A1 (exemple 1 : protocole d'obtention de microcompartiments cellulaires à partir de cellules humaines induites à la pluripotence).

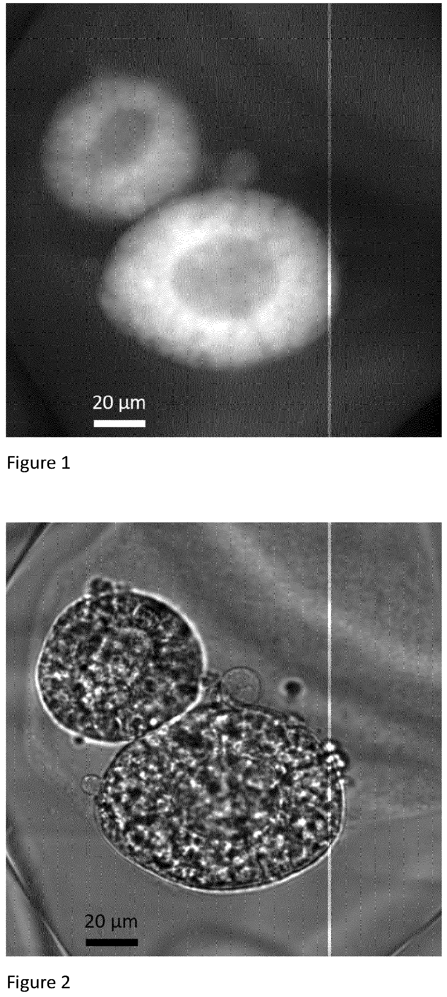

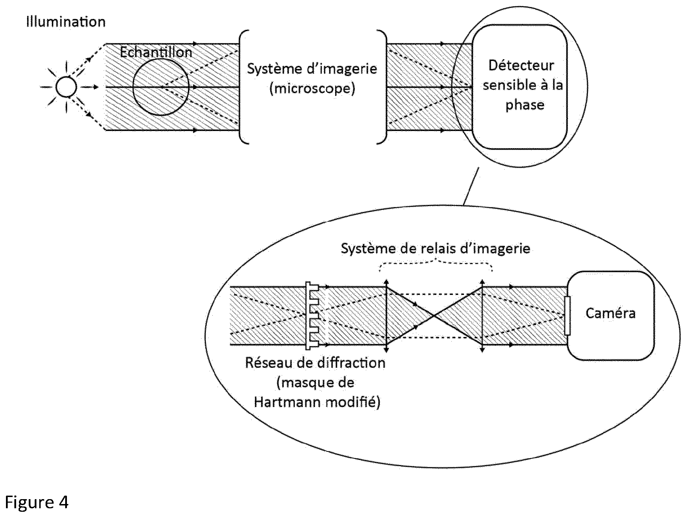

Un microcompartiment a été analysé selon un procédé d'imagerie par mesure de l'intensité, tel que décrit dans (Bon P. et al, Quadriwave lateral shearing interferometry for quantitative phase microscopy of living cells, 2009 Optical Society of America). L'intensité a été obtenue par démodulation des basses fréquences *via* un traitement dans l'espace de Fourier d'un interférogramme obtenu avec le protocole schématisé sur la Figure 4. Les résultats obtenus sont présentés sur la Figure 2.

Un microcompartiment a été analysé selon un procédé selon l'invention. Le protocole opératoire est décrit en suivant : une lumière halogène est utilisée pour illuminer en transmission l'échantillon, un objectif de microscope (x20, ouverture numérique 0.5) monté sur un microscope inversé est utilisé pour former l'image de l'échantillon sur un interféromètre autoréférencé (détecteur sensible entre autres à la phase). La technique d'imagerie utilisée est l'imagerie des gradients du front d'onde et en particulier la méthode de Hartmann modifié. Le protocole est également schématisé sur la Figure 4. La Figure 4 présente le système d'illumination, l'échantillon, le microscope et le détecteur sensible à la phase. Un zoom est présenté pour décrire le montage de Hartmann modifié utilisé pour obtenir les Figures 1 et 2.

Les résultats obtenus sont présentés sur la Figure 1.

On constate que l'image obtenue avec le procédé selon l'invention permet de mesurer la densité locale de l'échantillon et de la décorréler de l'absorption en comparaison avec l'image obtenue par la technique de l'art antérieur. En particulier, le procédé selon l'invention a permis de mesurer :
- les dimensions totales de chaque micro-tissu qui sont de 91µm (pour le plus gros) et 59µm (pour le plus petit) et calculées en mesurant le diamètre en pixels de chaque micro-tissu sur l'image D. En connaissant le grandissement total du système d'imagerie g_{y} et la taille physique d'un pixel mesure *Tpix,* la dimension d'un micro-tissu est alors de *D x Tpix* / *g_{y}.*
- Le diamètre de la zone de lumière au centre de chaque micro-tissu par une analyse des dimensions de la zone présentant une granulosité homogène et un déphase plus faible (i.e. image plus sombre) au centre du micro-tissu, mesurée à 38µm pour le micro-tissu le plus gros et 25µm pour le plus petit.
- la masse sèche de l'objet (l'intégrale sur l'objet de la masse volumique) qui est de 39µg pour le micro-tissu le plus gros et 16µg pour le plus petit, calculée comme décrit dans la publication (Ak-noun S. et al., Living cell dry mass measurement using quantitative phase imaging with quadriwave lateral shearing interferometry: an accuracy and sensitivity discussion, J/ of Biomedical Optics, 2015). Brièvement il s'agit de détourer automatiquement chaque micro-tissu par détermination des bords, d'évaluer la valeur de phase du fond par fit polynomial, de la soustraire à l'image, de sommer toute l'information de la phase de chaque micro-tissu et de convertir cela en masse par l'équation suivante : *m* = 0.18*pg* /µ*m*³ × ∫∫*_{microtissu} phase ·* d*s*
- le nombre de cellules qui est de 608±176 cellules pour le micro-tissu le plus gros et de 244±64 cellules pour le micro-tissu le plus petit. Cette valeur est obtenue par deux approches complémentaires. En connaissant la taille moyenne d'une cellule (5µm) et le volume de la zone où il y a des cellules dans le micro-tissu (volume du micro-tissu - volume de la lumière) on est déduit une valeur du nombre de cellules (respectivement 421 et 180 cellules). En connaissant la masse moyenne d'une cellule souche(50pg) et la masse totale du micro-tissu on en déduit une autre évaluation du nombre de cellules (respectivement 784 et 312 cellules).

### Exemple 2

Dans cet exemple, le procédé porte sur l'analyse de plusieurs micro-tissu humains contenus dans des microcompartiments, tel que décrit à l'exemple 1 de la demande WO2018/096277 A1 (exemple 1 : protocole d'obtention de microcompartiments cellulaires à partir de cellules humaines induites à la pluripotence). Des cellules souches pluripotentes humaines (Gibco Human Episomal IPSC) ont été encapsulées dans une matrice extracellulaire entourée par une paroi poreuse d'alginate et cultivées pendant 6 jours dans un milieu de croissance dans du milieu de croissance (MTesr1 suplémenté).

Les micro-tissus ont été analysés selon le procédé selon l'invention. Le protocole opératoire est décrit en suivant : une lumière halogène est utilisée pour illuminer en transmission l'échantillon, un objectif de microscope (x20 NA, ouverture numérique 0.45) monté sur un microscope inversé est utilisé pour former l'image de l'échantillon sur un interféromètre autoréférencé (détecteur sensible entre autres à la phase). La technique d'imagerie utilisée est l'imagerie de phase quantitative par interférométrie. Le protocole est également schématisé sur la Figure 4. L'ouverture numérique d'illumination est de 0,13 (cohérence spatiale de l'illumination), la longueur d'onde d'illumination est de 550 ± 100 nm (étendue spectrale de l'illumination ou cohérence temporelle de l'illumination).

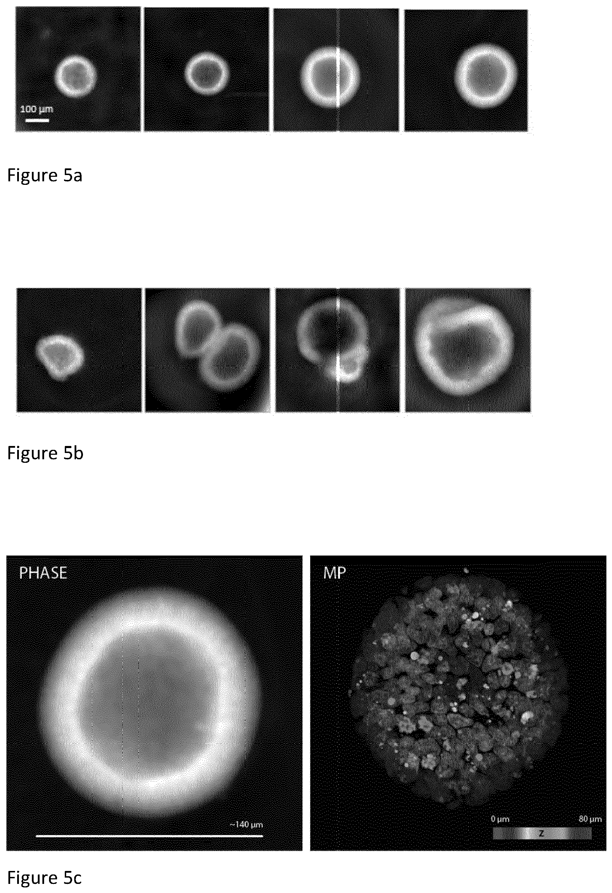

Les résultats obtenus sont présentés sur les Figures 5a, 5b et 5c. Ils permettent de caractériser la texture et la rondeur des microcompartiments avec des micro-tissus qui sont acceptables (5a) et non acceptables (5b).

Sur la Figure 5a les micro-tissus sont composés de cellules souches formant un cyste unique et répondant à des critères d'homogénéité de distribution de cellules et de rondeur permettant de les classer dans la catégorie des micro-tissus acceptables.

Sur la Figure 5b les micro-tissus sont composés de cellules souches formant plusieurs cystes et/ou présentant une inhomogénéité de distribution de cellules et de rondeur du permettant de les classer dans la catégorie des micro-tissus non-acceptables.

On constate que le procédé selon l'invention permet par analyse statistique de rondeur et d'homogénéité de texture de caractériser les micro-tissu acceptables et des micro-tissus non-acceptables même denses.

La Figure 5c, à gauche représente une d'un micro-tissu obtenu selon le procédé de l'exemple 2, et à droite représente le même micro-tissus acquis à l'aide de microscopie multiphotonique (les noyaux des cellules sont marqués par 10 ug/mL Hoechst 33342 (Thermofisher)). Environ la moitié des cellules sont visibles en multiphotonique comparé à l'invention. Le nombre total des cellules est de 500 environ.

On constate également que le marquage des noyaux des cellules des micro-tissus permet de corréler les données de la texture et la rondeur avec le nombre de cellules.

### Exemple 3

Dans cet exemple, le procédé porte sur l'analyse d'un micro-tissu humain contenu dans un microcompartiment, tel que décrit à l'exemple 1 de la demande WO2018/096277 A1 (exemple 1 : protocole d'obtention de microcompartiments cellulaires à partir de cellules humaines induites à la pluripotence) comparativement à un même microcompartiment sans micro-tissu.

Les microcompartiments avec et sans micro-tissus ont été analysés selon le procédé selon l'invention. Le protocole opératoire est décrit en suivant : une lumière halogène est utilisée pour illuminer en transmission l'échantillon, un objectif de microscope (x20 NA, ouverture numérique 0.45) monté sur un microscope inversé est utilisé pour former l'image de l'échantillon sur un interféromètre autoréférencé (détecteur sensible entre autres à la phase). La technique d'imagerie utilisée est l'imagerie de phase quantitative par interférométrie. L'ouverture numérique d'illumination est de 0,13 (cohérence spatiale de l'illumination), la longueur d'onde d'illumination est de 550 ± 100 nm (étendue spectrale de l'illumination ou cohérence temporelle de l'illumination).

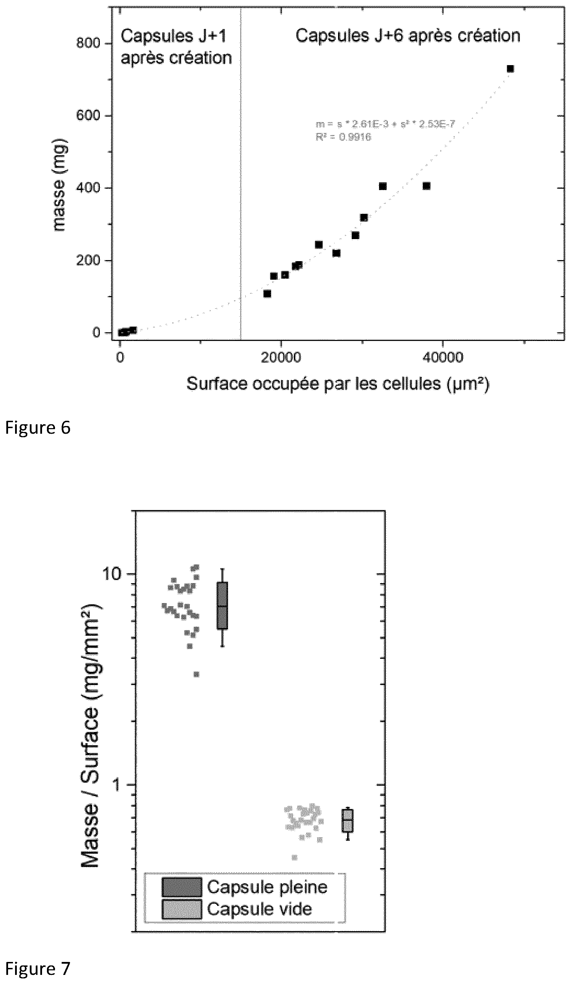

Les résultats obtenus sont présentés sur la Figure 6 et sur la Figure 7.

Sur la Figure 6 on voit parfaitement une évolution quadratique de la croissance des cellules et une dispersion à une date donnée pour déterminer la croissance de la population et trier les micro-tissus à maturité.

Sur la Figure 7 on constate que l''imagerie de phase permet de caractériser la densité des cellules présentes dans un micro-tissu.

## Revendications

1. Procédé de caractérisation in vitro d'un micro-tissu biologique d'eucaryote vivant congelé ou non congelé dont la plus petite dimension est supérieure ou égale à 20 micromètres et dont la plus grande dimension est inférieure ou égale à 1 cm, en imagerie par une technique de mesure de la phase sans faisceau de référence, l'étendue spectrale de l'illumination étant d'au minimum 5 nm dans le visible et au maximum 600 nm, et la cohérence spatiale de l'éclairage étant telle que l'ouverture numérique d'illumination est d'au minimum 5% et au maximum 90% de l'ouverture numérique du système d'imagerie, ledit procédé comprenant au moins l'étude de l'organisation des cellules dans le micro-tissu.

2. Procédé de caractérisation in vitro d'un micro-tissu selon la précédente revendication, **caractérisé en ce que** l'étude de l'organisation des cellules dans le micro-tissu comprend l'étude de la topologie du micro-tissu et/ou le positionnement relatif des cellules dans le micro-tissu.

3. Procédé de caractérisation in vitro d'un micro-tissu selon l'une des précédentes revendications, **caractérisé en ce que** la cohérence spatiale et/ ou temporelle de l'éclairage est sélectionnée de telle manière à ce que le contraste de la tavelure générée par le micro-tissu est inférieur à 75% du contraste maximum unitaire.

4. Procédé de caractérisation in vitro d'un micro-tissu selon la revendication 1, le micro-tissu étant un micro-tissu humain, animal ou végétal.

5. Procédé de caractérisation in vitro d'un micro-tissu selon l'une des précédentes revendications, **caractérisé en ce que** la plus grande dimension est inférieure ou égale à 1 mm.

6. Procédé de caractérisation in vitro d'un micro-tissu selon l'une des précédentes revendications, **caractérisé en ce que** la technique de mesure de la phase sans faisceau de référence est choisie parmi :
- l'analyse de front d'onde
- la modulation dynamique de la phase ou de l'intensité lumineuse dans la pupille du système d'illumination ou d'imagerie
- l'imagerie multiple d'intensité lumineuse avec modification du plan de mise au point.

7. Procédé de caractérisation in vitro d'un micro-tissu selon l'une des précédentes revendications, **caractérisé en ce que** la technique de mesure de la phase sans faisceau de référence est l'analyse de front d'onde et **en ce qu'**elle est réalisée à l'aide de l'imagerie des gradients du front d'onde.

8. Procédé de caractérisation in vitro d'un micro-tissu selon la précédente revendication, **caractérisée en ce que** l'imagerie des gradients du front d'onde est choisie parmi la méthode Shack-Hartmann, Hartmann modifié ou non, le partitionnement de la pupille et l'imagerie de champ de speckle.

9. Procédé de caractérisation in vitro d'un micro-tissu selon l'une des revendications 1 à 5, **caractérisé en ce que** la technique de mesure de la phase sans faisceau de référence est la modulation dynamique de la phase ou de l'intensité lumineuse dans la pupille du système d'illumination ou d'imagerie et **en ce qu'**elle est réalisée à l'aide de la technique de ptychographie ou de la modulation de phase sélective de certaines fréquences dans la pupille.

10. Procédé de caractérisation in vitro d'un micro-tissu selon l'une des revendications 1 à 5, **caractérisé en ce que** la technique de mesure de la phase sans faisceau de référence est l'imagerie multiple d'intensité lumineuse avec modification du plan de mise au point et **en ce qu'**elle est réalisée à l'aide de l'imagerie multiplan simultané ou séquentiel.

11. Procédé de caractérisation in vitro d'un micro-tissu selon l'une des précédentes revendications, **caractérisé en ce qu'**il comprend la mesure de la phase et de l'intensité lumineuse de la lumière ayant traversé le micro-tissu.

12. Procédé de caractérisation in vitro d'un micro-tissu selon l'une des précédentes revendications, **caractérisé en ce qu'**il est réalisé en ligne sur le contenu d'un bioréacteur.

13. Procédé de caractérisation in vitro d'un micro-tissu selon l'une des revendications 1 à 12, à **caractérisé en ce qu'**il est réalisé :
i) en cellules de flux, ou
ii) en prélèvement ponctuel hors bioréacteur, ou
iii) pour trier les micro-tissus en ligne ou hors ligne.

14. Procédé de caractérisation in vitro d'un micro-tissu selon l'une des précédentes revendications, **caractérisé en ce qu'**il comprend la mesure :
- de la densité du micro-tissu, à partir de la mesure de la phase, et
- éventuellement de l'absorption locale du micro-tissu, à partir de la mesure de la phase et de la mesure de l'intensité lumineuse de la lumière ayant traversé le micro-tissu.

15. Procédé de caractérisation in vitro d'un micro-tissu selon l'une des précédentes revendications, **caractérisé en ce qu'**il comprend la mesure d'au moins un des paramètres suivants :
- dimensions du micro-tissu,
- dimensions d'au moins une des cellules du micro-tissu,
- nombre de cellules dans le micro-tissu,
- masse globale et locale du micro-tissu,
- densité globale et locale du micro-tissu,
- distribution de masse dans le micro-tissu,
- topologie du micro-tissu
- positionnement relatif des cellules dans le micro-tissu,
- viabilité des cellules du micro-tissu,
- texture du micro-tissu.

16. Procédé de caractérisation in vitro d'un micro-tissu selon l'une des précédentes revendications, **caractérisé en ce que** le micro-tissu est encapsulé dans un microcompartiment comprenant une couche externe en hydrogel.

17. Procédé de caractérisation in vitro d'un micro-tissu selon l'une des précédentes revendications, **caractérisé en ce que** le micro-tissu se présente sous la forme d'un ovoïde, d'un tuboïde, d'un sphéroïde ou d'une sphère, ou de monocouches partiellement repliées sur elles-mêmes (2,5D).

18. Procédé de caractérisation in vitro d'un micro-tissu selon l'une des précédentes revendications, **caractérisé en ce que** le micro-tissu est entouré au moins partiellement d'une matrice extracellulaire.

19. Procédé de caractérisation in vitro d'un micro-tissu selon l'une des précédentes revendications, **caractérisé en ce que** le micro-tissu est un micro-tissu biologique humain ou animal destiné à être greffé chez l'homme ou l'animal.

20. Procédé de caractérisation in vitro d'un micro-tissu selon l'une des précédentes revendications, **caractérisé en ce que** le micro-tissu est un micro-tissu biologique humain ou animal choisi parmi les micro-tissus épithéliaux, conjonctifs, musculaires ou nerveux.

21. Procédé de caractérisation in vitro d'un micro-tissu selon l'une des précédentes revendications, **caractérisé en ce que** le micro-tissu comprend des cellules différenciées cardiaques ou des cellules rétiniennes ou des cellules neurales ou des cellules du foie ou des chondrocytes ou des kératinocytes ou des cellules lymphoïdes, ou des cellules souches hématopoiétiques ou des cellules souches mésenchymateuses ou des cellules pluripotentes sous la forme d'un épiblaste.

22. Procédé de caractérisation in vitro d'un micro-tissu selon l'une des précédentes revendications, **caractérisé en ce que** le micro-tissu est un micro-tissu produit à des fins de bioproduction de médicament ou alimentaire.

23. Procédé de caractérisation in vitro d'un micro-tissu selon l'une des revendications 1 à 19, **caractérisé en ce que** le micro-tissu est un micro-tissu biologique végétal choisi parmi les méristèmes, les parenchymes, les tissus conducteurs, les tissus de soutien, les tissus de revêtement ou de protection, les tissus de sécrétion et les tissus nourriciers.

24. Utilisation d'un procédé de caractérisation in vitro d'un micro-tissu selon l'une des précédentes revendications, pour :
- contrôler la qualité d'un micro-tissu, et/ou
- mesurer l'augmentation de la biomasse d'un micro-tissu pendant sa culture et/ou son amplification, et/ou
- suivre la différenciation et/ou l'organisation d'un micro-tissu durant sa maturation, et/ou
- déterminer le phénotype de cellules du micro-tissu, et/ou
- confirmer l'absence de cellules non différenciées dans le micro-tissu, et/ou
- déterminer la viabilité des cellules du micro-tissu.

25. Utilisation d'un procédé de caractérisation in vitro d'un micro-tissu selon l'une des revendications 1 à 19, pour le screening d'embryon obtenu par fécondation in vitro, l'embryon étant le micro-tissu.

## Patentansprüche

1. Verfahren zur In-vitro-Charakterisierung eines gefrorenen oder nicht gefrorenen biologischen Mikrogewebes von lebenden Eukaryoten, dessen kleinste Abmessung größer oder gleich 20 Mikrometer ist und dessen größte Abmessung kleiner oder gleich 1 cm ist, in der Bildgebung durch eine Technik zum Messen der Phase ohne Referenzstrahl, wobei die spektrale Ausdehnung der Beleuchtung mindestens 5 nm im sichtbaren Bereich und höchstens 600 nm beträgt, und die räumliche Kohärenz der Beleuchtung derart ist, dass die numerische Apertur der Beleuchtung mindestens 5 % und höchstens 90 % der numerischen Apertur des Bildgebungssystems beträgt, wobei das Verfahren mindestens die Untersuchung der Organisation der Zellen im Mikrogewebe umfasst.

2. Verfahren zur In-vitro-Charakterisierung eines Mikrogewebes nach dem vorstehenden Anspruch, **dadurch gekennzeichnet, dass** die Untersuchung der Organisation der Zellen in dem Mikrogewebe die Untersuchung der Topologie des Mikrogewebes und/oder die relative Positionierung der Zellen in dem Mikrogewebe umfasst.

3. Verfahren zur In-vitro-Charakterisierung eines Mikrogewebes nach einem der vorstehenden Ansprüche, **dadurch gekennzeichnet, dass** die räumliche und/oder zeitliche Kohärenz der Beleuchtung so gewählt wird, dass der vom Mikrogewebe erzeugte Specklemusterkontrast geringer als 75 % des maximalen Einheitskontrastes ist.

4. Verfahren zur In-vitro-Charakterisierung eines Mikrogewebes nach Anspruch 1, wobei das Mikrogewebe ein menschliches, tierisches oder pflanzliches Mikrogewebe ist.

5. Verfahren zur In-vitro-Charakterisierung eines Mikrogewebes nach einem der vorstehenden Ansprüche, **dadurch gekennzeichnet, dass** die größte Abmessung kleiner oder gleich 1 mm ist.

6. Verfahren zur In-vitro-Charakterisierung eines Mikrogewebes nach einem der vorstehenden Ansprüche, **dadurch gekennzeichnet, dass** die Technik des Messens der Phase ohne Referenzstrahl ausgewählt wird aus:
- der Wellenfrontanalyse
- der dynamischen Modulation der Phase oder der Lichtintensität in der Pupille des Beleuchtungs- oder Abbildungssystems
- der multiplen Bildgebung der Lichtintensität mit Modifikation der Fokussierungsebene.

7. Verfahren zur In-vitro-Charakterisierung eines Mikrogewebes nach einem der vorstehenden Ansprüche, **dadurch gekennzeichnet, dass** die Technik zum Messen der Phase ohne Referenzstrahl die Wellenfrontanalyse ist **und dass** sie mittels der Bildgebung der Wellenfrontgradienten durchgeführt wird.

8. Verfahren zur In-vitro-Charakterisierung eines Mikrogewebes nach dem vorstehenden Anspruch, **dadurch gekennzeichnet, dass** die Bildgebung der Wellenfrontgradienten aus der Shack-Hartmann-Methode, modifizierter oder nicht modifizierter Hartmann-Methode, der Pupillenaufteilung und der Speckle-Feld-Bildgebung ausgewählt wird.

9. Verfahren zur In-vitro-Charakterisierung eines Mikrogewebes nach einem der Ansprüche 1 bis 5, **dadurch gekennzeichnet, dass** die Technik zum Messen der Phase ohne Referenzstrahl die dynamische Modulation der Phase oder der Lichtintensität in der Pupille des Beleuchtungs- oder Abbildungssystems ist **und dass** sie mittels der Ptychographie-Technik oder der selektiven Phasenmodulation bestimmter Frequenzen in der Pupille durchgeführt wird.

10. Verfahren zur In-vitro-Charakterisierung eines Mikrogewebes nach einem der Ansprüche 1 bis 5, **dadurch gekennzeichnet, dass** die Technik zum Messen der Phase ohne Referenzstrahl die multiple Bildgebung der Lichtintensität mit Modifikation der Fokussierungsebene ist **und dass** sie mittels simultaner oder sequenzieller Multiplan-Bildgebung durchgeführt wird.

11. Verfahren zur In-vitro-Charakterisierung eines Mikrogewebes nach einem der vorstehenden Ansprüche, **gekennzeichnet dadurch, dass** es die Messung der Phase und der Lichtintensität des Lichts, das das Mikrogewebe durchdrungen hat, umfasst.

12. Verfahren zur In-vitro-Charakterisierung eines Mikrogewebes nach einem der vorstehenden Ansprüche, **dadurch gekennzeichnet, dass** es online am Inhalt eines Bioreaktors durchgeführt wird.

13. Verfahren zur In-vitro-Charakterisierung eines Mikrogewebes nach einem der Ansprüche 1 bis 12, **dadurch gekennzeichnet, dass** es durchgeführt wird:
i) in Flusszellen, oder
ii) bei punktueller Probenahme außerhalb des Bioreaktors, oder
iii) zum Sortieren der Mikrogewebe online oder offline.

14. Verfahren zur In-vitro-Charakterisierung eines Mikrogewebes nach einem der vorstehenden Ansprüche, **dadurch gekennzeichnet, dass** es die Messung umfasst:
- der Dichte des Mikrogewebes, ausgehend von der Messung der Phase, und
- gegebenenfalls der lokalen Absorption des Mikrogewebes, ausgehend von der Messung der Phase und der Messung der Lichtintensität des Lichts, das das Mikrogewebe durchdrungen hat.

15. Verfahren zur In-vitro-Charakterisierung eines Mikrogewebes nach einem der vorstehenden Ansprüche, **dadurch gekennzeichnet, dass** es die Messung mindestens eines der folgenden Parameter umfasst:
- Abmessungen des Mikrogewebes,
- Abmessungen mindestens einer der Zellen des Mikrogewebes,
- Anzahl der Zellen im Mikrogewebe,
- globale und lokale Masse des Mikrogewebes,
- globale und lokale Dichte des Mikrogewebes,
- Massenverteilung im Mikrogewebe,
- Topologie des Mikrogewebes
- relative Positionierung der Zellen in dem Mikrogewebe,
- Lebensfähigkeit der Zellen des Mikrogewebes,
- Textur des Mikrogewebes.

16. Verfahren zur In-vitro-Charakterisierung eines Mikrogewebes nach einem der vorstehenden Ansprüche, **dadurch gekennzeichnet, dass** das Mikrogewebe in einem Mikrokompartiment eingekapselt ist, das eine äußere Schicht aus Hydrogel umfasst.

17. Verfahren zur In-vitro-Charakterisierung eines Mikrogewebes nach einem der vorstehenden Ansprüche, **dadurch gekennzeichnet, dass** das Mikrogewebe in Form eines Ovoids, eines Tuboids, eines Sphäroids oder einer Sphäre oder von teilweise auf sich selbst zurückgefalteten Monoschichten (2,5D) vorliegt.

18. Verfahren zur In-vitro-Charakterisierung eines Mikrogewebes nach einem der vorstehenden Ansprüche, **dadurch gekennzeichnet, dass** das Mikrogewebe mindestens teilweise von einer extrazellulären Matrix umgeben ist.

19. Verfahren zur In-vitro-Charakterisierung eines Mikrogewebes nach einem der vorstehenden Ansprüche, **dadurch gekennzeichnet, dass** das Mikrogewebe ein menschliches oder tierisches biologisches Mikrogewebe ist, das zur Transplantation beim Menschen oder Tier bestimmt ist.

20. Verfahren zur In-vitro-Charakterisierung eines Mikrogewebes nach einem der vorstehenden Ansprüche, **dadurch gekennzeichnet, dass** das Mikrogewebe ein menschliches oder tierisches biologisches Mikrogewebe ist, das aus Epithel-, Bindegewebs-, Muskel- oder Nervenmikrogeweben ausgewählt ist.

21. Verfahren zur In-vitro-Charakterisierung eines Mikrogewebes nach einem der vorstehenden Ansprüche, **dadurch gekennzeichnet, dass** das Mikrogewebe differenzierte Herzzellen oder Netzhautzellen oder Nervenzellen oder Leberzellen oder Chondrozyten oder Keratinozyten oder lymphoide Zellen oder hämatopoetische Stammzellen oder mesenchymale Stammzellen oder pluripotente Zellen in Form eines Epiblasten umfasst.

22. Verfahren zur In-vitro-Charakterisierung eines Mikrogewebes nach einem der vorstehenden Ansprüche, **dadurch gekennzeichnet, dass** das Mikrogewebe ein Mikrogewebe ist, das zu Zwecken der Bioproduktion von Arzneimitteln oder Nahrungsmitteln hergestellt wird.

23. Verfahren zur In-vitro-Charakterisierung eines Mikrogewebes nach einem der Ansprüche 1 bis 19, **dadurch gekennzeichnet, dass** das Mikrogewebe ein pflanzliches biologisches Mikrogewebe ist, das aus Meristemen, Parenchymen, leitenden Geweben, Stützgeweben, Hüll- oder Schutzgeweben, Sekretionsgeweben und Nährgeweben ausgewählt ist.

24. Verwendung eines Verfahrens zur In-vitro-Charakterisierung eines Mikrogewebes nach einem der vorstehenden Ansprüche, zum:
- Kontrollieren der Qualität eines Mikrogewebes, und/oder
- Messen der Zunahme der Biomasse eines Mikrogewebes während seiner Kultur und/oder seiner Amplifikation, und/oder
- Verfolgen der Differenzierung und/oder Organisation eines Mikrogewebes während seiner Reifung, und/oder
- Bestimmen des Phänotyps von Zellen des Mikrogewebes, und/oder
- Bestätigen des Fehlens von nicht differenzierten Zellen in dem Mikrogewebe, und/oder
- Bestimmen der Lebensfähigkeit der Zellen des Mikrogewebes.

25. Verwendung eines Verfahrens zur In-vitro-Charakterisierung eines Mikrogewebes nach einem der Ansprüche 1 bis 19, für das Screening von durch In-vitro-Befruchtung erhaltenem Embryo, wobei der Embryo das Mikrogewebe ist.

## Claims

1. Method for in vitro characterization of a frozen or unfrozen living eukaryotic biological microtissue of which the smallest dimension is greater than or equal to 20 micrometers and of which the largest dimension is less than or equal to 1 cm, by imaging using a reference-beam-free phase measurement technique, the spectral range of the illumination being a minimum of 5 nm in the visible range and a maximum of 600 nm, and the spatial coherence of the lighting being such that the illumination numerical aperture is a minimum of 5% and a maximum of 90% of the numerical aperture of the imaging system, said method comprising at least the study of the organization of the cells in the microtissue.

2. Method for in vitro characterization of a microtissue according to the preceding claim, **characterized in that** the study of the organization of the cells in the microtissue comprises the study of the topology of the microtissue and/or the relative positioning of the cells in the microtissue.

3. Method for in vitro characterization of a microtissue according to either of the preceding claims, **characterized in that** the spatial and/or temporal coherence of the lighting is selected in such a way that the contrast of the speckle generated by the microtissue is less than 75% of the maximum unit contrast.

4. Method for in vitro characterization of a microtissue according to claim 1, the microtissue being a human, animal or plant microtissue.

5. Method for in vitro characterization of a microtissue according to any of the preceding claims, **characterized in that** the largest dimension is less than or equal to 1 mm.

6. Method for in vitro characterization of a microtissue according to any of the preceding claims, **characterized in that** the reference-beam-free phase measurement technique is selected from:
- wavefront analysis
- dynamic modulation of the phase or luminous intensity in the pupil of the illumination or imaging system
- multiple luminous-intensity imaging with modification of the plane of focus.

7. Method for in vitro characterization of a microtissue according to any of the preceding claims, **characterized in that** the reference-beam-free phase measurement technique is wavefront analysis **and in that** it is performed using wavefront gradient imaging.

8. Method for in vitro characterization of a microtissue according to the preceding claim, **characterized in that** wavefront gradient imaging is selected from the Shack-Hartmann, modified or unmodified Hartmann, pupil partitioning and speckle field imaging methods.

9. Method for in vitro characterization of a microtissue according to any of claims 1 to 5, **characterized in that** the reference-beam-free phase measurement technique is the dynamic modulation of the phase or luminous intensity in the pupil of the illumination or imaging system **and in that** it is performed using the ptychography technique or the selective phase modulation of certain frequencies in the pupil.

10. Method for in vitro characterization of a microtissue according to any of claims 1 to 5, **characterized in that** the reference-beam-free phase measurement technique is multiple luminous-intensity imaging with modification of the plane of focus **and in that** it is performed using simultaneous or sequential multi-planar imaging.

11. Method for in vitro characterization of a microtissue according to any of the preceding claims, **characterized in that** it comprises measurement of the phase and luminous intensity of the light having passed through the microtissue.

12. Method for in vitro characterization of a microtissue according to any of the preceding claims, **characterized in that** it is performed online on the contents of a bioreactor.

13. Method for in vitro characterization of a microtissue according to any of claims 1 to 12, **characterized in that** it is performed:
i) in flow cells, or
ii) by spot sampling outside the bioreactor, or
iii) to sort microtissues online or offline.

14. Method for in vitro characterization of a microtissue according to any of the preceding claims, **characterized in that** it comprises measuring:
- the density of the microtissue, based on the phase measurement, and
- optionally, the local absorption of the microtissue, based on the phase measurement and measurement of the luminous intensity of the light having passed through the microtissue.

15. Method for in vitro characterization of a microtissue according to any of the preceding claims, **characterized in that** it comprises measuring at least one of the following parameters:
- dimensions of the microtissue,
- dimensions of at least one of the microtissue cells,
- number of cells in the microtissue,
- global and local mass of the microtissue,
- global and local density of the microtissue,
- mass distribution in the microtissue,
- topology of the microtissue
- relative positioning of the cells in the microtissue,
- viability of the microtissue cells,
- texture of the microtissue.

16. Method for in vitro characterization of a microtissue according to any of the preceding claims, **characterized in that** the microtissue is encapsulated in a microcompartment comprising an outer hydrogel layer.

17. Method for in vitro characterization of a microtissue according to any of the preceding claims, **characterized in that** the microtissue is in the form of an ovoid, tuboid, spheroid or sphere, or of partially folded monolayers (2,5D).

18. Method for in vitro characterization of a microtissue according to any of the preceding claims, **characterized in that** the microtissue is at least partially surrounded by an extracellular matrix.

19. Method for in vitro characterization of a microtissue according to any of the preceding claims, **characterized in that** the microtissue is a human or animal biological microtissue intended for grafting in humans or animals.

20. Method for in vitro characterization of a microtissue according to any of the preceding claims, **characterized in that** the microtissue is a human or animal biological microtissue selected from epithelial, connective, muscular or nervous microtissues.

21. Method for in vitro characterization of a microtissue according to any of the preceding claims, **characterized in that** the microtissue comprises cardiac differentiated cells or retinal cells or neural cells or liver cells or chondrocytes or keratinocytes or lymphoid cells, or hematopoietic stem cells or mesenchymal stem cells or pluripotent cells in the form of an epiblast.

22. Method for in vitro characterization of a microtissue according to any of the preceding claims, **characterized in that** the microtissue is a microtissue produced for drug or food bioproduction purposes.

23. Method for in vitro characterization of a microtissue according to any of claims 1 to 19, **characterized in that** the microtissue is a plant biological microtissue selected from meristems, parenchyma, conductive tissues, support tissues, coating or protective tissues, secretory tissues and feeder tissues.

24. Use of a method for in vitro characterization of a microtissue according to any of the preceding claims, for:
- controlling the quality of a microtissue, and/or
- measuring the increase in biomass of a microtissue during cultivation and/or amplification, and/or
- monitoring the differentiation and/or organization of a microtissue during maturation, and/or
- determining the phenotype of microtissue cells, and/or
- confirming the absence of undifferentiated cells in the microtissue, and/or
- determining the viability of the microtissue cells.

25. Use of a method for in vitro characterization of a microtissue according to any of claims 1 to 19, for screening an embryo obtained by in vitro fertilization, the embryo being the microtissue.
